# EUROPEAN PATENT APPLICATION

(11) **EP 3 646 860 A1**
(43) Date of publication of application: **06.05.2020**
(21) Application number: 18306425.2
(22) Date of filing: 31.10.2018
(51) Int. Cl.: A61K 31/195, A61K 9/00, A61P 29/00

(54) **BACLOFEN FOR USE IN THE TOPICAL TREATMENT OF LOCALIZED MUSCULOSKELETAL PAIN**

(71) Applicant: ASSISTANCE PUBLIQUE, HOPITAUX DE PARIS, 75004 Paris (FR)
(72) Inventor: GRECO, Céline, 75012 PARIS (FR)
(74) Representative: Gevers & Orès

(57) **Abstract**

The present invention relates to Baclofen and pharmaceutically acceptable salts thereof, or a composition comprising such a compound for use in the topical treatment and prevention of localized musculoskeletal pain.

## Description

The present invention relates to Baclofen and pharmaceutically acceptable salts thereof, or a composition comprising such a compound, for use in the topical treatment and prevention of localized musculoskeletal pain.

Musculoskeletal pain, which can be localized in joints (arthralgia), entheses (enthesalgia), tendons (tendonalgia) and muscles (myalgia), is commonly found in many patients. For example, in patients suffering from hemophilia, repeated bleedings in joints cause severe pain in the joints which are subject to major stress, such as ankles, knees, hips and elbows. Patients suffering from juvenile rheumatoid arthritis, rheumatoid arthritis, psoriatic arthritis, ankylosis spondylitis, ostheoarthritis or mastocytosis often have pain in entheses and/or joints causing mobility disorders. Patients suffering from myopathy such as Carnitine palmitoyltransferase II deficiency or FSH dystrophy complain on burning pain in muscles, spasms muscles and severe pain from changes in posture and strain on remaining muscles.

Generally, pain is relieved using a variety of anti-inflammatory drugs (such as nonsteroidal anti-inflammatory drugs "NSAIDs") or painkiller drugs (such as aspirin, paracetamol, weak or strong opioids ...). However, these treatments are at times ineffective and some can have many systemic side effects. For example, anti-inflammatory drugs, which are often used as the first-line treatment, highly increase the risk of bleeding and are thus proscribed for treating hemophiliac patients. They are also forbidden in case of renal insufficiency, peptic ulcer, cardiovascular diseases, inflammatory bowel diseases, etc.... In addition, topical administration of conventional NSAIDs has been known to be ineffective because only a therapeutically ineffective amount of the drug penetrates the skin, and patients with acne, psoriasis or eczema are typically refractory to topical or oral administration of NSAIDs.

There thus remains a genuine need for an alternative and effective treatment having no or a less side effects compared with actual treatments.

The present invention is believed to meet such need by providing a topical composition for treating or preventing localized musculoskeletal pain.

Baclofen of formula (I) is a muscle relaxant considered the first-line treatment for spasticity.

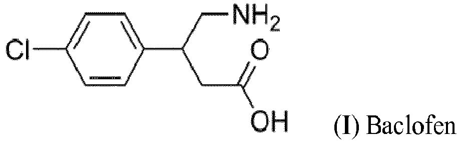

When administrated orally (*per os*)*,* Baclofen works pre- and post-synaptically as a gamma aminobutylic acid (GABA) B agonist at the spinal level and binds to its receptors, leading to membrane hyperpolarization. This molecule restricts calcium influx, which subsequently (i) restricts endogenous excitatory neurotransmitters from being released and (ii) inhibits mono- and polysynaptic spinal reflexes.

The Inventors have in a surprising manner demonstrated the efficacy of Baclofen in the topical treatment and prevention of musculoskeletal pain localized in joints, entheses, tendons and/or muscles.

Thus, the present invention concerns a compound selected from Baclofen and pharmaceutically acceptable salts thereof, for use in the topical treatment or prevention of a localized musculoskeletal pain, in particular arthralgia, enthesalgia, tendonalgia and/or myalgia.

The present invention further relates to a topical composition comprising a compound according to the invention as active ingredient and at least one pharmaceutically acceptable excipient, for use in the treatment or prevention of a localized musculoskeletal pain, in particular arthralgia, enthesalgia, tendonalgia and/or myalgia.

The present invention further relates to a method for treating and/or preventing a localized musculoskeletal pain, in particular arthralgia, enthesalgia, tendonalgia and/or myalgia by means of topical administration, to a patient in need thereof, of an effective amount of a compound or composition according to the invention.

The present invention further relates to the use of a compound or composition according to the invention for the manufacture of a topical medication for treating and/or preventing localized musculoskeletal pain, in particular arthralgia, enthesalgia, tendonalgia and/or myalgia.

"Baclofen" as used herein (also known via its brand name Lioresal®) refers to the molecule β-(4-chlorophenyl)-γ-aminobutyric acid, enantiomers, racemic mixtures, polymorphs, salts, solvates, esters or hydrates thereof. Baclofen includes R-baclofen (D-baclofen), S-baclofen (L-baclofen), or their mixtures including the racemate. The racemate refers to a mixture of R and S-baclofen (DL-baclofen) in equal proportions.

In the present invention, "pharmaceutically acceptable" is intended to mean that which is useful in the preparation of a pharmaceutical composition, generally safe, nontoxic and neither biologically nor otherwise undesirable and acceptable for both veterinary and human pharmaceutical use.

"Pharmaceutically acceptable salt" of a compound is intended to mean a salt that is pharmaceutically acceptable, as defined herein, and that has the desired pharmacological activity of the parent compound.

Salts of baclofen include salts of acidic or basic groups present in compounds of the application. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, or pamoate salts. Certain compounds of the invention can form pharmaceutically acceptable salts with various amino acids. Suitable base salts include, but are not limited to, aluminum, calcium, lithium, magnesium, potassium, sodium, zinc, or diethanolamine salts. Potassium salts include potassium chloride, potassium bicarbonate, potassium phosphate, gluconate, potassium citrate, or the like.

In the present invention, the term "musculoskeletal pain" designates pain that affects the joints, muscles, entheses, ligaments and/or tendons.

In the present invention, "arthralgia" is intended to mean joint pain. This joint pain can be caused by various diseases, disorders or injuries. In the invention, arthralgia is not, is partially or is totally caused by inflammation.

In the present invention, "myalgia" is intended to mean muscle pain. This muscle pain can be caused by various diseases, disorders or injuries. In the invention, myalgia is not, is partially or is totally caused by inflammation. In particular embodiments, myalgia is or is not associated to an enthesitis, *i.e.* an inflammation of the entheses (*i.e.* the sites where tendons or ligaments insert into the bone).

In the present invention, "enthesalgia" is intended to mean entheses pain. Entheses pain can be caused by various diseases, disorders or injuries. In the invention, enthesalgia is not, is partially or is totally caused by inflammation. Enthesopathies are disorders of peripheral ligamentous or muscular attachments. For example, entheses pain occurs in spondoarthropathy such as ankylosing spondylitis, plantar fascitis, and Achilles tendinitis, adhesive capsulitis of shoulder, rotator cuff syndrome of shoulder and allied disorders, periarthritis of shoulder, scapulohumeral fibrositi, enthesopathy of elbow region, enthesopathy of wrist and carpus, Bursitis of hand or wrist, periarthritis of wrist, enthesopathy of hip region, bursitis of hip, gluteal tendinitis, iIliac crest spur, Psoas tendinitis, trochanteric tendinitis, enthesopathy of knee, enthesopathy of ankle, tarsus and calcaneous, other peripheral enthesopathies.

In the present invention, "tendonalgia" is intended to mean tendon pain. Tendons are the fibrous tissues that connect muscle to bone. Tendonalgia can be caused by various diseases, disorders or injuries. In the invention, tendonalgia is not, is partially or is totally caused by inflammation. Recent basic science research suggests that little or no inflammation is present in these conditions. Thus, traditional treatment modalities aimed at controlling inflammation such as corticosteroid injections and nonsteroidal anti-inflammatory medications (NSAIDS) may not be efficient options.

By "treatment" is meant, according to the present invention, the inhibition of the development of, more particularly the regression of, preferably the disappearance of a musculoskeletal pain according to the invention.

By "prevention" is meant, according to the present invention, to prevent or delay the appearance of a musculoskeletal pain according to the invention.

The treatment or prevention according to the invention applies to humans or animals.

By "topical" is meant, according to the present invention, that the compound or composition according to the invention will be administered by application on the skin surface or the mucous membranes.

The topical composition according to the invention may in particular be in any form allowing topical application, such as a cream, a gel, an ointment, a solution, a lotion, a spray, an aerosol spray, an aerosol foam or a patch. Preferably, the composition according to the invention will be in cream or gel form.

In an embodiment, the topical composition of the invention can be applied to the skin by an applicator, such as a roll-on, a stick, an impregnated wipe or an impregnated glove.

In an embodiment, the composition of the invention can also be dispensed from a pump pack or from an aerosol container.

In an embodiment, the topical composition of the invention comprises Baclofen in a concentration from 5% to 25%, in particular from 6% to 25%, more particularly from 10% to 25%, by weight relative to the weight of the final composition.

In an embodiment, the topical composition of the invention comprises Baclofen in a concentration of 5%, 6%, 7%, 8%, 9%, 10%, 11%, 12%, 13%, 14%, 15%, 16%, 17%, 18%, 19% , 20%, 21%, 22%, 23% , 24%, or 25% by weight relative to the weight of the final composition.

In an embodiment, the topical composition of the invention is formulated into unit dose form.

The compound or topical composition according to the invention will be administered one or more times per day, the duration being variable according to the pain intensity and easily adjustable by the person skilled in the art or the practitioner.

In an embodiment, the topical composition of the invention is applied to the affected area once daily, twice daily, three times daily, once every second day, three times weekly, twice weekly or once weekly.

By "pharmaceutically acceptable excipient" is meant, according to the invention, an excipient that is compatible with the other ingredients of the composition and that produces no adverse effect, allergic reaction or other undesirable reaction when it is administered to a human or an animal.

According to the invention, by "excipient" is meant in particular one or more surfactants, for example macrogols, hydroxy stearates, ethoxylated fatty acid esters, ethoxylated fatty alcohols; one or more solvents, such as for example octyldodecanol, propylene glycol dicaprylocaprate; one or more hydrosoluble polymers, for example PVP, hyaluronic acid or sodium hyaluronate; one or more thickeners such as for example natural or semisynthetic gums; one or more gelling agents, for example carbomers; one or more inorganic fillers, for example zinc oxide, talc, clays; one or more emulsifiers such as cetearyl alcohol; one or more preservatives, for example phenoxyethanol; one or more antibacterials; one or more antiseptics; one or more antioxidants, for example tocopherol acetate; one or more chelating agents, for example EDTA; one or more pigments; one or more fragrances; one or more colorants; one or more pH adjustors such as salts, acids, bases; or a mixture thereof.

In an embodiment, the topical composition of the invention further comprises a penetration enhancer.

By "penetration enhancer" is meant, according to the invention, a chemical substance that promotes the transdermal penetration of the drug applied at the surface of the skin. For example, a penetration enhancer can be selected among alcohols, amides, esters, glycols, fatty acids, pyrrolidones, sulfoxides, surfactants, terpenes, urea, cyclodextrins, water, vitamin E or phospholipids.

In an embodiment, the topical composition further comprises a base allowing transdermal diffusion of said compound. In particular, such a base may be selected from the group comprising Pentravan®, Pentravan® Plus, Phytobase®, Lipovan® and Pluronic Lecithin Organogel (PLO), preferably Pentravan®.

In an embodiment, the topical composition of the invention does not contain any compound with analgesic and/or anti-inflammatory effects.

In an embodiment, the topical composition is administered to a patient to treat and/or prevent musculoskeletal pain due to juvenile rheumatoid arthritis, rheumatoid arthritis, psoriatic arthritis, ankylosis spondylitis, ostheoarthritis, spondyloarthritis, sacroiliac joint osteoarthritis and osteitis condensans ilii, diffuse and sacroiliac pain in joint hypermobility in case of Elhers-Danlos disease, hypo- and hyperparathyroidism, primary Sjögren's syndrome; sacroiliitis of connective tissue diseases such as lupus, skeletal manifestations of Behçet's disease, relapsing polychondritis, and sarcoidosis, axial joint involvement of Whipple's disease, rheumatism associated with pityriasis rubra-pilaris, PAPA and PASS syndromes, fibrodysplasia ossificans progressiva, paraneoplastic spondyloarthritis, hypophosphatemia, calcineurin inhibitor-induced pain syndromes (CIPS), skeletal manifestations of fluorosis, pachydermoperiostosis and joint involvement in adult Kawasaki disease, chikungunya chronic arthritis, lyme disease, fibromyalgia, systemic mastocytosis, complex regional pain syndrome, hemophilia, Facioscapulohumeral muscular dystrophy, Ankylosing Spondylarthritis, metabolic myalgia, mastocytosis, knee arthropathy, Sever's disease, stress myalgia, Ankylosing Spondylarthritis, calcaneum enthesitis, cervical myalgia, rhabdomyolysis, myalgia post-chemotherapy or Floating-Harbor syndrom.

In an embodiment, the topical composition is administered to a patient to treat and/or prevent musculoskeletal pain due to fibromyalgia or systemic mastocytosis.

In an embodiment, the topical composition is administered to a patient to treat and/or prevent musculoskeletal pain due to a complex regional pain syndrome.

In an embodiment, the topical composition is administered to a patient to treat and/or prevent musculoskeletal pain due to hemophilia.

In an embodiment, the topical composition is administered to a patient to treat and/or prevent musculoskeletal pain, in particular chronic arthralgia and myalgia, due to Hepatitis C virus or HIV infection.

In an embodiment, the topical composition is administered to a patient to treat and/or prevent joint, muscular, entheses and/or tendon pain due to muscular hyper-excitability and spasms due to multiple sclerosis, myopathies, facioscapulohumeral muscular dystrophy, hereditary periodic fever syndromes, dengue, metabolic myopathies, genetic disorders such as CPT2 deficiency, Duchenne muscular dystrophy, etc..., episodic myalgia, dermatomyositis, myotoxicity of statins, guillain-barre syndrome.

In an embodiment, the topical composition is administered to a patient to treat and/or prevent musculoskeletal pain induced by chemotherapy (for example Taxanes) or haemopathies (for example waldenstrom).

In an embodiment, the topical composition is administered to a patient to treat and/or prevent musculoskeletal pain induced by facioscapulohumeral muscular dystrophy. juvenile spondyloarthritis, metabolic myopathy, systemic mastocytosis, haemophilia with ankle, elbow and knee pain, Ehler Danlos syndrome or arthralgia and enthesalgia of knee.

In an embodiment, the topical composition is administered to a patient to treat and/or prevent musculoskeletal pain induced by Facioscapulohumeral muscular dystrophy, Ankylosing Spondylarthritis, metabolic myalgia, mastocytosis, hemophilia, knee arthropathy, Ehler Danlos syndrom, Sever's disease, stress myalgia, Ankylosing Spondylarthritis, calcaneum enthesitis, cervical myalgia, rhabdomyolysis, myalgia post-chemotherapy or Floating-Harbor syndrom.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### EXAMPLES

19 patients were treated with success with topical applications of Baclofen cream.

The cream was prepared with Baclofen powder mixed with a Pentravan® Base (Fagron) at a final concentration of 10 % w/w.

The intensity of pain was rated using a Visual Analogue Scale (VAS) from 1 to 10.

Results are shown in Table 1.

All patients report a pain decrease from -3 to -7 units on VAS.

| **Patient** | **Age (years)** | **Sex** | **Disease** | **Type of pain** | **VAS pain before application (/10)** | **VAS pain after application (/10)** | **VAS at one month for patients with regular application (/10)** | **Type of application** |
|---|---|---|---|---|---|---|---|---|
| **n°1** | 15 | M | Facioscapulohumeral muscular dystrophy | muscular pain | 7 | 0 | 2 to 3 | twice a day + in case of pain during the day |
| **n°2** | 40 | F | Facioscapulohumeral muscular dystrophy | muscular pain | 6 | 0 | 2 to 3 | twice a day + in case of pain during the day |
| **n°3** | 12 | F | Ankylosing Spondylarthritis | tendonalgia + enthesalgia | 5 | 2 | | in case of pain |
| **n°4** | 6 | M | metabolic myalgia | muscular pain | 6 | 0 | | in case of pain |
| **n°5** | 45 | F | mastocytosis | tendonalgia + muscular pain | 7 | 2 | | in case of pain |
| **n°6** | 46 | F | mastocytosis | tendonalgia + muscular pain | 8 | 2 | 5 | twice a day |
| **n°7** | 65 | M | hemophilia | arthralgia | 8 | 2 | 3 | twice a day |
| **n°8** | 23 | F | knee arthropathy | arthralgia | 6 | 1 | 2 | twice a day |
| **n°9** | 44 | F | Ehler Danlos syndrom | tendonalgia + muscular pain | 7 | 2 | 2 | twice a day |
| **n°10** | 14 | F | Sever's disease | enthesalgia | 6 | 1 | | in case of pain |
| **n°11** | 12 | F | stress myalgia | muscular pain | 6 | 1 | | in case of pain |
| **n°12** | 12 | F | Ankylosing Spondylarthritis | tendonalgia | 6 | 2 | 3 | twice a day |
| n°13 | 66 | F | calcaneum enthesitis | enthesalgia | 5 | 2 | 3 | twice a day |
| **n°14** | 10 | F | cervical myalgia | muscular pain | 6 | 0 | | in case of pain |
| **n°15** | 35 | M | Ankylosing Spondylarthritis | tendonalgia + enthesalgia | 5 | 2 | | in case of pain |
| **n°16** | 35 | M | rhabdomyolysis | muscular pain | 4 | 1 | | in case of pain |
| **n°17** | 65 | M | myalgia post-chemotherapy | muscular pain | 6 | 0 | | in case of pain |
| **n°18** | 75 | F | myalgia post-chemotherapy | muscular pain | 6 | 0 | 0 | twice a day |
| **n°19** | 15 | M | Floating-Harbor syndrom | muscular pain | 7 | 0 | 2 | twice a day + in case of pain during the day |

## Claims

1. A compound selected from Baclofen and pharmaceutically acceptable salts thereof, for use in the topical treatment or prevention of a localized musculoskeletal pain.

2. A topical composition comprising a compound according to claim 1 as active ingredient, and at least one pharmaceutically acceptable excipient, for use in the treatment or prevention of a localized musculoskeletal pain.

3. The topical composition for use according to claim 2, wherein the composition is in the form of a solution, a gel, a cream, an ointment, a lotion, a spray, an aerosol spray, an aerosol foam or a patch.

4. The topical composition for use according to any one of claims 2 and 3, wherein the compound is in a concentration from 5% to 25%, preferably in a concentration of 10%, by weight relative to the weight of the final composition.

5. The topical composition for use according to any one of claims 2 to 4, wherein the topical composition is formulated into unit dose form.

6. The topical composition for use according to any one of claims 2 to 5, wherein the topical composition does not contain any compound with analgesic and/or anti-inflammatory effects.

7. The topical composition for use according to any one of claims 2 to 6, wherein the topical composition is applied to the affected area once daily, twice daily, three times daily, once every second day, three times weekly, twice weekly or once weekly.

8. The topical composition for use according to any one of claims 2 to 7, further comprising a penetration enhancer.

9. The topical composition for use according to any one of claims 2 to 8, wherein the topical composition is administered to a patient to treat and/or prevent a musculoskeletal pain due to juvenile rheumatoid arthritis, rheumatoid arthritis, psoriatic arthritis, ankylosis spondylitis, ostheoarthritis, spondyloarthritis, sacroiliac joint osteoarthritis and osteitis condensans ilii, diffuse and sacroiliac pain in joint hypermobility in case of Elhers-Danlos disease, hypo- and hyperparathyroidism, primary Sjögren's syndrome; sacroiliitis of connective tissue diseases such as lupus, skeletal manifestations of Behçet's disease, relapsing polychondritis, and sarcoidosis, axial joint involvement of Whipple's disease, rheumatism associated with pityriasis rubra-pilaris, PAPA and PASS syndromes, fibrodysplasia ossificans progressiva, paraneoplastic spondyloarthritis, hypophosphatemia, calcineurin inhibitor-induced pain syndromes (CIPS), skeletal manifestations of fluorosis, pachydermoperiostosis and joint involvement in adult Kawasaki disease, chikungunya chronic arthritis, lyme disease, fibromyalgia, systemic mastocytosis, complex regional pain syndrome, hemophilia, Facioscapulohumeral muscular dystrophy, Ankylosing Spondylarthritis, metabolic myalgia, mastocytosis, knee arthropathy, Sever's disease, stress myalgia, Ankylosing Spondylarthritis, calcaneum enthesitis, cervical myalgia, rhabdomyolysis, myalgia post-chemotherapy or Floating-Harbor syndrom.

10. The topical composition for use according to any one of claims 2 to 8, wherein the topical composition is administered to a patient to treat and/or prevent musculoskeletal pain induced by Facioscapulohumeral muscular dystrophy, Ankylosing Spondylarthritis, metabolic myalgia, mastocytosis, hemophilia, knee arthropathy, Ehler Danlos syndrom, Sever's disease, stress myalgia, Ankylosing Spondylarthritis, calcaneum enthesitis, cervical myalgia, rhabdomyolysis, myalgia post-chemotherapy or Floating-Harbor syndrom.
